# EUROPEAN PATENT APPLICATION

(11) **EP 3 643 790 A1**
(43) Date of publication of application: **29.04.2020**
(21) Application number: 18819599.4
(22) Date of filing: 20.06.2018
(51) Int. Cl.: C12Q 1/68, C12N 15/09, C12Q 1/686, C12Q 1/6876

(54) **METHOD FOR NUCLEIC ACID DETECTION, PRIMER FOR NUCLEIC ACID DETECTION, AND KIT FOR NUCLEIC ACID DETECTION**

(30) Priority: 23.06.2017 JP 2017123247
(71) Applicant: Eiken Kagaku Kabushiki Kaisha, Taito-ku Tokyo 110-8408 (JP)
(72) Inventor: MICHIYUKI, Satoru, Ohtawara-shi Tochigi 324-0036 (JP)
(74) Representative: J A Kemp LLP
(86) International application number: PCT/JP2018/023537
(87) International publication number: WO 2018/235886

(57) **Abstract**

[Problem] An objective of the present invention is to develop a new type of method for nucleic acid detection that can be suitably used for detection of short nucleic acids.

[Solution] The present invention provides a method for nucleic acid detection including
i) hybridizing a detection target nucleic acid 1 and a primer for nucleic acid detection 3 to form a DNA-RNA complex,
ii) removing a first polynucleotide segment 301 from the primer for nucleic acid detection 3 by degrading at least a portion of the DNA-RNA complex with a nuclease 4, and
iii) hybridizing the primer for nucleic acid detection 3 to a template nucleic acid 2 to perform a reaction for amplifying the template nucleic acid 2 by a polymerase 5.

Sequence Listing
SEQUENCE LISTING
<110> EIKEN KAGAKU KABUSHIKI KAISHA
<120> Method for detecting nucleic acid, primer for detecting nucleic

acid and kit for detecting nucleic acid

<130> E01P17001PCT

<160> 10

<170> PatentIn version 3.5

<210> 1
<211> 18
<212> DNA
<213> Artificial Sequence

<220>
<223> ART7-F3 primer

<400> 1
ggacaatcga agccagaa
18

<210> 2
<211> 18
<212> DNA
<213> Artificial Sequence

<220>
<223> ART7-B3 primer

<400> 2
atcacggatc gtatgtgg 18

<210> 3
<211> 34
<212> DNA
<213> Artificial Sequence

<220>
<223> ART7-FIP primer

<400> 3
cgcttggata gtcggatgca agggtcaatg gtac 34

<210> 4
<211> 33
<212> DNA
<213> Artificial Sequence

<220>
<223> ART7-BIP primer

<400> 4
acggtgtatg cttcggtgtg cgaacctatc agc 33

<210> 5
<211> 50
<212> DNA
<213> Artificial Sequence

<220>
<223> ART7-BIP-D

<220>
<221> misc_feature
<222> (50)..(50)
<223> phosphorylated

<400> 5
acggtgtatg cttcggtgtg cgaacctatc agcccatgag attcaacagt 50

<210> 6
<211> 50
<212> DNA
<213> Artificial Sequence

<220>
<223> ART7-BIP-R

<220>
<221> misc_feature
<222> (34)..(49)
<223> RNA

<220>
<221> misc_feature
<222> (50)..(50)
<223> phosphorylated

<400> 6
acggtgtatg cttcggtgtg cgaacctatc agcccaugag auucaacagt 50

<210> 7
<211> 18
<212> DNA
<213> Artificial Sequence

<220>
<223> ART7-LF

<400> 7
gctagctaag tgccatcc 18

<210> 8
<211> 18
<212> DNA
<213> Artificial Sequence

<220>
<223> ART7-LB primer

<400> 8
aacgatcgca ctaagcat 18

<210> 9
<211> 600
<212> DNA
<213> Artificial Sequence

<220>

<223>    ARITA artificial gene

<400>    9
attcgaaggg tgattggatc ggagatagga tgggtcaatc gtagggacaa tcgaagccag    60

aatgcaaggg tcaatggtac gcagaatgga tggcacttag ctagccagtt aggatccgac    120

tatccaagcg tgtatcgtac ggtgtatgct tcggagtaac gatcgcacta agcatggctc    180

aatcctaggc tgataggttc gcacatagca tgccacatac gatccgtgat tgctagcgtg    240

attcgtaccg agaactcacg ccttatgact gcccttatgt caccgcttat gtctcccgat    300

atcacacccg ttatctcagc cctaatctct gcggtttagt ctggccttaa tccatgcctc    360

atagctaccc tcataccatc gctcatacct tccgacattg catccgtcat tccaaccctg    420

attcctacgg tctaacctag cctctatcct acccagttag gttgcctctt agcatccctg    480

ttacgtacgc tcttaccatg cgtcttacct tggcactatc gatgggagta tggtagcgag    540

tatggaacgg actaacgtag gcagtaagct agggtgtaag gttgggacta aggatgccag    600

<210>    10
<211>    72
<212>    DNA
<213>    Mammal

<220>
<221>    misc_feature

<223>    miR-21(Pre-mature)

<400>    10

tgtcgggtag cttatcagac tgatgttgac tgttgaatct catggcaaca ccagtcgatg    60

ggctgtctga    ca

72

SEQUENCE LISTING

<110>  EIKEN KAGAKU KABUSHIKI KAISHA

<120>  Method for detecting nucleic acid, primer for detecting nucleic
       acid and kit for detecting nucleic acid

<130>  E01P17001PCT

<160>  10

<170>  PatentIn version 3.5

<210>  1
<211>  18
<212>  DNA
<213>  Artificial Sequence

<220>
<223>  ART7-F3 primer

<400>  1
ggacaatcga agccagaa                                                    18


<210>  2
<211>  18
<212>  DNA
<213>  Artificial Sequence

<220>
<223>  ART7-B3 primer

<400>  2
atcacggatc gtatgtgg                                                    18


<210>  3
<211>  34
<212>  DNA
<213>  Artificial Sequence

<220>
<223>  ART7-FIP primer

<400>  3
cgcttggata gtcggatgca agggtcaatg gtac                                  34


<210>  4
<211>  33
<212>  DNA
<213>  Artificial Sequence

<220>
<223>  ART7-BIP primer

<400>  4
acggtgtatg cttcggtgtg cgaacctatc agc                                   33


<210>  5
<211>  50

<212>  DNA
<213>  Artificial Sequence

<220>
<223>  ART7-BIP-D



<220>
<221>  misc_feature
<222>  (50)..(50)
<223>  phosphorylated

<400>  5
acggtgtatg cttcggtgtg cgaacctatc agcccatgag attcaacagt                50


<210>  6
<211>  50
<212>  DNA
<213>  Artificial Sequence

<220>
<223>  ART7-BIP-R



<220>
<221>  misc_feature
<222>  (34)..(49)
<223>  RNA

<220>
<221>  misc_feature
<222>  (50)..(50)
<223>  phosphorylated

<400>  6
acggtgtatg cttcggtgtg cgaacctatc agcccaugag auucaacagt                50


<210>  7
<211>  18
<212>  DNA
<213>  Artificial Sequence

<220>
<223>  ART7-LF

<400>  7
gctagctaag tgccatcc                                                   18


<210>  8
<211>  18
<212>  DNA
<213>  Artificial Sequence

<220>
<223>  ART7-LB primer

<400>  8
aacgatcgca ctaagcat                                                   18

<210>  9
<211>  600
<212>  DNA
<213>  Artificial Sequence

<220>
<223>  ARITA artificial gene

<400>  9
attcgaaggg tgattggatc ggagatagga tgggtcaatc gtagggacaa tcgaagccag      60

aatgcaaggg tcaatggtac gcagaatgga tggcacttag ctagccagtt aggatccgac     120

tatccaagcg tgtatcgtac ggtgtatgct tcggagtaac gatcgcacta agcatggctc     180

aatcctaggc tgataggttc gcacatagca tgccacatac gatccgtgat tgctagcgtg     240

attcgtaccg agaactcacg ccttatgact gcccttatgt caccgcttat gtctcccgat     300

atcacacccg ttatctcagc cctaatctct gcggtttagt ctggccttaa tccatgcctc     360

atagctaccc tcataccatc gctcatacct tccgacattg catccgtcat tccaaccctg     420

attcctacgg tctaacctag cctctatcct acccagttag gttgcctctt agcatccctg     480

ttacgtacgc tcttaccatg cgtcttacct tggcactatc gatgggagta tggtagcgag     540

tatggaacgg actaacgtag gcagtaagct agggtgtaag gttgggacta aggatgccag     600


<210>  10
<211>  72
<212>  DNA
<213>  Mammal


<220>
<221>  misc_feature
<223>  miR-21(Pre-mature)

<400>  10
tgtcgggtag cttatcagac tgatgttgac tgttgaatct catggcaaca ccagtcgatg      60
ggctgtctga ca                                                          72

## Description

### Technical Field

The present invention relates to a method for nucleic acid detection for detecting a detection target nucleic acid utilizing a principle of nucleic acid amplification such as a LAMP method or a PCR method. The present invention relates to a method for nucleic acid detection that is particularly suitable for detection of a short nucleic acid of from 10 to 100 bases and can be used for detection of miRNA or the like.

The present invention relates to a primer for nucleic acid detection and a kit for nucleic acid detection.

### Background Art

There are a large amount of short nucleic acids that are not translated into proteins in cells. Although these short nucleic acids were thought to have no function, research after the 1990s has revealed that they have an important role in living organisms.

Among these short nucleic acids, short nucleic acids called miRNA (microRNA) have been found to play an important role in regulation of gene expression. A miRNA is a single-stranded RNA of from 20 to 25 bases, and as a precursor thereof, a miRNA gene on a chromosome is transcribed with an RNA polymerase to produce a pri-miRNA (primary miRNA) containing a hairpin structure, and then pri-miRNA is cleaved by Drosha which is a nuclear RNase to produce a pre-miRNA (precursor miRNA) of 60-70 base having a hairpin structure. A pre-miRNA is transported from inside a nucleus to cytoplasm and cleaved with Dicer which is an RNase in cytoplasm, to form a double-stranded RNA (miRNA duplex) having from 21 to 24 bases. A double-stranded RNA is incorporated into Ago protein, and only one RNA strand (mature miRNA) eventually forms a stable complex with the Ago protein to form an RNA-induced silencing complex (RISC). A RISC regulates gene expression by binding to a target mRNA having a partially complementary sequence to a mature miRNA incorporated into the RISC and suppressing translation of the target mRNA.

Although miRNAs play important roles in cell function by regulating expression of a large number of target genes, it has also become clear that miRNAs are closely related to diseases such as cancer. Previous studies have reported miRNAs that show abnormal expression in a variety of diseases such as cancer, and miRNAs are expected to be developed as diagnostic tools for a variety of diseases.

However, as a method for detecting miRNA, a microarray method is often used, but there are problems such as complicated operation and high cost.

As a method for detecting a miRNA using a nucleic acid amplification method, a method of detecting a miRNA by polyadenylating the miRNA and adding a poly-A sequence, then synthesizing a cDNA by reverse transcription using a primer containing bases complementary to a poly-T sequence and the miRNA, and performing nucleic acid amplification of the cDNA using a primer complementary to this cDNA has been developed (Patent Document 1).

A method for detecting a miRNA by hybridizing the miRNA to a primer having a 3' region specific to the miRNA and a stem loop, performing a primer extension reaction, and performing an amplification reaction using the extended primer as a template has also been developed (Patent Document 2).

Incidentally, ribonuclease (RNase) is an example of an enzyme that degrades RNA. A method of utilizing RNase active at high temperatures to start a PCR reaction hot by removing a blocking group when a primer to which a blocking group that prevents primer extension is bound is double-stranded with a template nucleic acid has been developed (Patent Document 3).

### Citation List

### Patent Documents

Patent Document 1 U.S. Patent Application Publication 2009/0220969
Patent Document 2 U.S. Patent No. 7575863
Patent Document 3 Japanese Patent No. 5539325

### Summary of Invention

### Technical Problem

Although, as a conventional method for detecting miRNA, microarray analysis is often used, there are problems such as complicated operation and high cost. Although miRNA detection methods using nucleic acid amplification have been developed, there have been problems in practicality in terms of specificity and simplicity.

In view of the above-described conventional situation, an object of the present invention is to develop a new type of method for nucleic acid detection that can be suitably used for detection of short nucleic acids.

### Solution to Problem

In order to solve the above-described problems, the present inventors have intensively studied to find that by using a special primer for nucleic acid detection, a portion of which forms a DNA-RNA complex by hybridization with a detection target nucleic acid and which is degraded by a nuclease, the primer for nucleic acid detection is activated and a nucleic acid amplification reaction occurs only when a detection target nucleic acid is present, thereby completing the present invention.

In short, the present invention provides the following first invention relating to a method for nucleic acid detection, the following second invention relating to a primer for nucleic acid detection, and the following third invention relating to a kit for nucleic acid detection.
(1) The first invention relates to a method for nucleic acid detection for detecting a detection target nucleic acid, using at least
   A) a template nucleic acid that is amplified and serves as a detection index,
   B) a primer for nucleic acid detection including a first polynucleotide segment having a base sequence complementary to at least a portion of the detection target nucleic acid and a second polynucleotide segment having a base sequence complementary to a portion of the template nucleic acid, wherein
      when the detection target nucleic acid is RNA, at least the 5' end of the first polynucleotide segment is DNA, and when the detection target nucleic acid is DNA, at least the 5' end of the first polynucleotide segment is RNA, and
      the second polynucleotide segment is linked to the 5' side of the first polynucleotide segment,
   C) a nuclease that degrades a DNA-RNA complex, and
   D) a polymerase, and including
      i) contacting a test sample containing the detection target nucleic acid with the primer for nucleic acid detection to hybridize the detection target nucleic acid and the primer for nucleic acid detection to form a DNA-RNA complex,
      ii) removing the first polynucleotide segment from the primer for nucleic acid detection by degrading at least a portion of the DNA-RNA complex with the nuclease, and
      iii) hybridizing the primer for nucleic acid detection to the template nucleic acid and amplifying the template nucleic acid with the polymerase.
(2) In the method for nucleic acid detection according to the first invention, preferably, the detection target nucleic acid is a short nucleic acid having a size of from 10 to 100 bases.
(3) In the method for nucleic acid detection according to any one of the above, the primer for nucleic acid detection may further include a third polynucleotide segment including the same base sequence as a portion of the base sequence of a 5' side region from a region complementary to the second polynucleotide segment in the template nucleic acid on the 5' side of the second polynucleotide segment.
(4) In the method for nucleic acid detection according to any one of the above,
   preferably, the detection target nucleic acid is RNA, and the nuclease is a nuclease that degrades DNA in the DNA-RNA complex.
(5) In the method for nucleic acid detection according to any one of the above,
   preferably, the 3' end of the primer for nucleic acid detection includes a modification that stops an amplification reaction.
(6) In the method for nucleic acid detection according to any one of the above, the primer for nucleic acid detection may further include a fourth polynucleotide segment that has a non-natural artificial base sequence on the 3' side of the first polynucleotide segment and does not hybridize with the template nucleic acid.
(7) In the method for nucleic acid detection according to any one of the above, preferably, in the above iii), nucleic acid amplification by a LAMP method or nucleic acid amplification by a PCR method.
(8) In the method for nucleic acid detection according to any one of the above, preferably, the template nucleic acid is a template nucleic acid having a non-natural artificial base sequence.
(9) In the method for nucleic acid detection according to any one of the above, the template nucleic acid includes a common base sequence identical to a portion of the detection target nucleic acid, and
   a portion on the 3' side of the second polynucleotide segment can be complementary to the common base sequence.
(10) The second invention relates to a primer for nucleic acid detection for detecting a detection target nucleic acid, including
   a first polynucleotide segment having a base sequence complementary to at least a portion of the detection target nucleic acid, and
   a second polynucleotide segment having a base sequence complementary to a portion of the template nucleic acid that is amplified and serves as a detection index, wherein
   when the detection target nucleic acid is RNA, at least the 5' end of the first polynucleotide segment is DNA, and when the detection target nucleic acid is DNA, at least the 5' end of the first polynucleotide segment is RNA, and
   the second polynucleotide segment is linked to the 5' side of the first polynucleotide segment.
(11) In the primer for nucleic acid detection according to the second invention, preferably, the first polynucleotide segment has a size of from 10 to 100 bases.
(12) In the primer for nucleic acid detection according to any one of the above, the primer for nucleic acid detection may further include a third polynucleotide segment including the same base sequence as a portion of the base sequence of a 5' side region from a region complementary to the second polynucleotide segment in the template nucleic acid on the 5' side of the second polynucleotide segment.
(13) In the primer for nucleic acid detection according to any one of the above, preferably, the 3' end of the primer for nucleic acid detection includes a modification that stops an amplification reaction.
(14) In the primer for nucleic acid detection according to any one of the above, the primer for nucleic acid detection may further include a fourth polynucleotide segment that has a non-natural artificial base sequence on the 3' side of the first polynucleotide segment and does not hybridize with the template nucleic acid.
(15) In the primer for nucleic acid detection according to any one of the above, a portion on the 3' side of the second polynucleotide segment can be made not only complementary to a part of the template nucleic acid but also complementary to a part of the detection target nucleic acid.
(16) The third invention relates to a kit for nucleic acid detection for detecting a detection target nucleic acid, including
   A) a template nucleic acid that is amplified and serves as a detection index,
   B) a primer for nucleic acid detection including a first polynucleotide segment having a base sequence complementary to at least a portion of the detection target nucleic acid and a second polynucleotide segment having a base sequence complementary to a portion of the template nucleic acid, wherein
      when the detection target nucleic acid is RNA, at least the 5' end of the first polynucleotide segment is DNA, and when the detection target nucleic acid is DNA, at least the 5' end of the first polynucleotide segment is RNA, and
      the second polynucleotide segment is linked to the 5' side of the first polynucleotide segment, and
   C) a nuclease that degrades a DNA-RNA complex.
(17) In the kit for nucleic acid detection according to the third invention, preferably, the first polynucleotide segment having a size of from 10 to 100 bases.
(18) In the kit for nucleic acid detection according to any one of the above, the primer for nucleic acid detection may further include a third polynucleotide segment including the same base sequence as a portion of the base sequence of a 5' side region from a region complementary to the second polynucleotide segment in the template nucleic acid on the 5' side of the second polynucleotide segment.
(19) In the kit for nucleic acid detection according to any one of the above, preferably, the detection target nucleic acid is RNA, and the nuclease is a nuclease that degrades DNA in the DNA-RNA complex.
(20) The kit for nucleic acid detection according to any one of the above, preferably, further includes D) polymerase.
(21) In the kit for nucleic acid detection according to any one of the above, the primer for nucleic acid detection may further include a fourth polynucleotide segment that has a non-natural artificial base sequence on the 3' side of the first polynucleotide segment and does not hybridize with the template nucleic acid.
(22) In the kit for nucleic acid detection according to any one of the above, the template nucleic acid includes a common base sequence identical to a portion of the detection target nucleic acid, and
   a portion on the 3' side of the second polynucleotide segment can be complementary to the common base sequence.

### Advantageous Effects of Invention

The present invention uses a primer for nucleic acid detection including a first polynucleotide segment having a base sequence complementary to at least a portion of a detection target nucleic acid and a second polynucleotide segment having a base sequence complementary to a portion of a template nucleic acid, and when the detection target nucleic acid is present, the detection target nucleic acid and a primer for nucleic acid detection are hybridized to form a DNA-RNA complex, and at least a part of the DNA-RNA complex is decomposed by a nuclease, whereby the first polynucleotide segment is removed and the primer for nucleic acid detection is activated. Then, the second polynucleotide segment of the primer for nucleic acid detection hybridizes to the template nucleic acid, and an amplification reaction of the template nucleic acid is started by a polymerase. As described above, according to the present invention, a normal amplification reaction of a template nucleic acid occurs only when a detection target nucleic acid is present, and therefore a detection target nucleic acid can be detected using amplification of a template nucleic acid as an index.

### Brief Description of Drawings

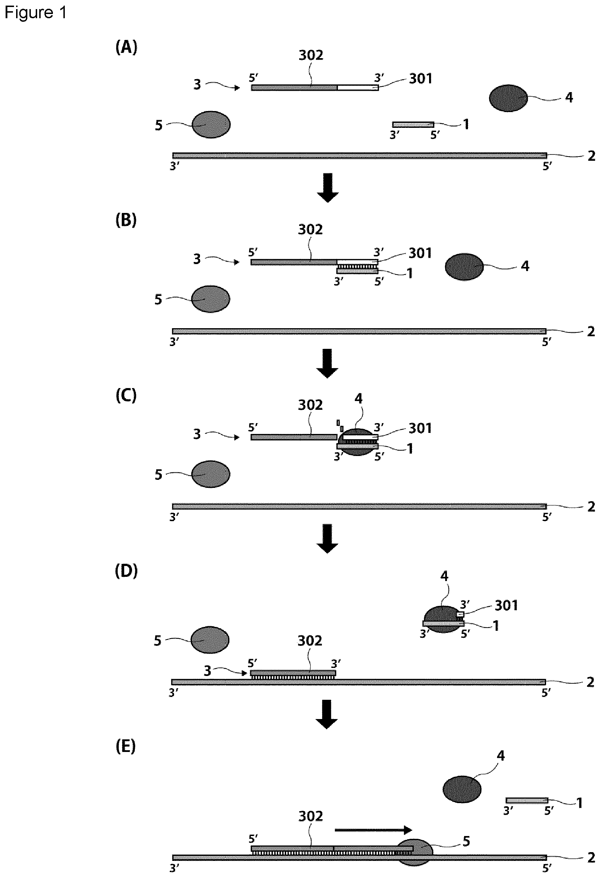

Fig. 1 is a schematic diagram showing a first embodiment of the method for nucleic acid detection of the present invention. Fig. 1 (A) shows a state in which a test sample containing a detection target nucleic acid and a reaction solution are mixed. Fig. 1 (B) shows a state in which the detection target nucleic acid and a first polynucleotide segment are hybridized to form a DNA-RNA complex. Fig. 1 (C) shows a state in which a first polynucleotide segment of the DNA-RNA complex is degraded by a nuclease. Fig. 1 (D) shows a state in which an activated primer for nucleic acid detection is hybridized to a template nucleic acid. Fig. 1 (E) shows a state in which the template nucleic acid is amplified by a polymerase.

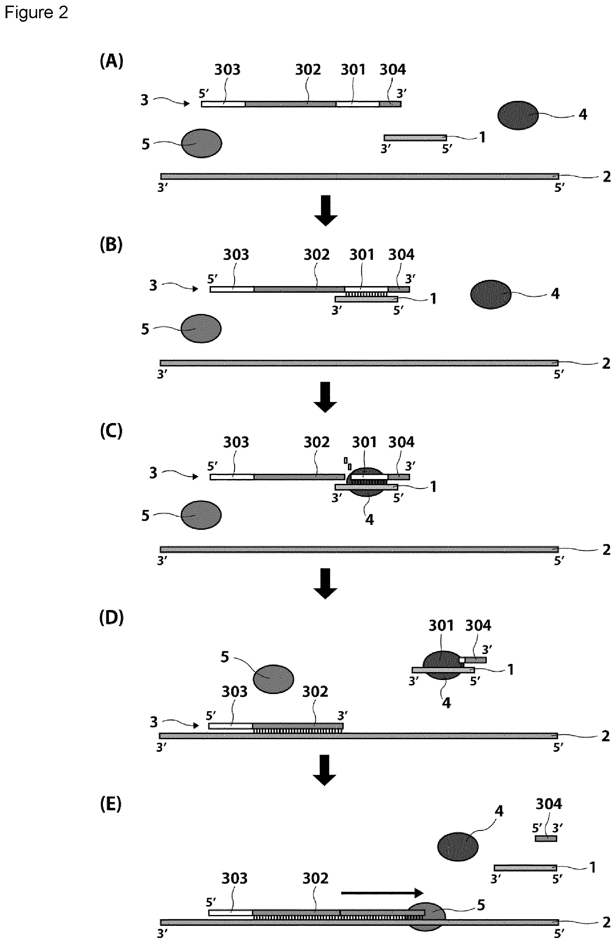

Fig. 2 is a schematic diagram showing a second embodiment of the method for nucleic acid detection of the present invention. Fig. 2 (A) shows a state in which a test sample containing a detection target nucleic acid and a reaction solution are mixed. Fig. 2 (B) shows a state in which the detection target nucleic acid and a first polynucleotide segment are hybridized to form a DNA-RNA complex. Fig. 2 (C) shows a state in which the first polynucleotide segment of the DNA-RNA complex is degraded by a nuclease. Fig. 2 (D) shows a state in which an activated primer for nucleic acid detection is hybridized to a template nucleic acid. Fig. 2 (E) shows a state in which the template nucleic acid is amplified by a polymerase.

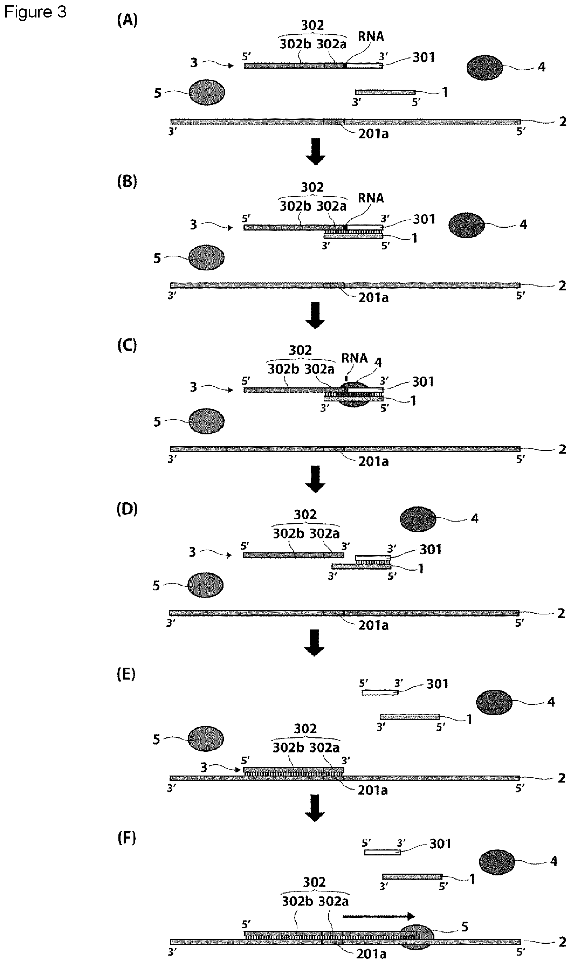

Fig. 3 is a schematic diagram showing a third embodiment of the method for nucleic acid detection of the present invention. Fig. 3 (A) shows a state in which a test sample containing a detection target nucleic acid and a reaction solution are mixed. Fig. 3 (B) shows a state in which the detection target nucleic acid and a primer for nucleic acid detection are hybridized to form a DNA-RNA complex. Fig. 3 (C) shows a state in which the first polynucleotide segment of the DNA-RNA complex is degraded by a nuclease. Fig. 3 (D) shows a state in which the first polynucleotide segment has been removed from the primer for nucleic acid detection. Fig. 3 (E) shows a state in which an activated primer for nucleic acid detection is hybridized to a template nucleic acid. Fig. 3 (F) shows a state in which the template nucleic acid is amplified by a polymerase.

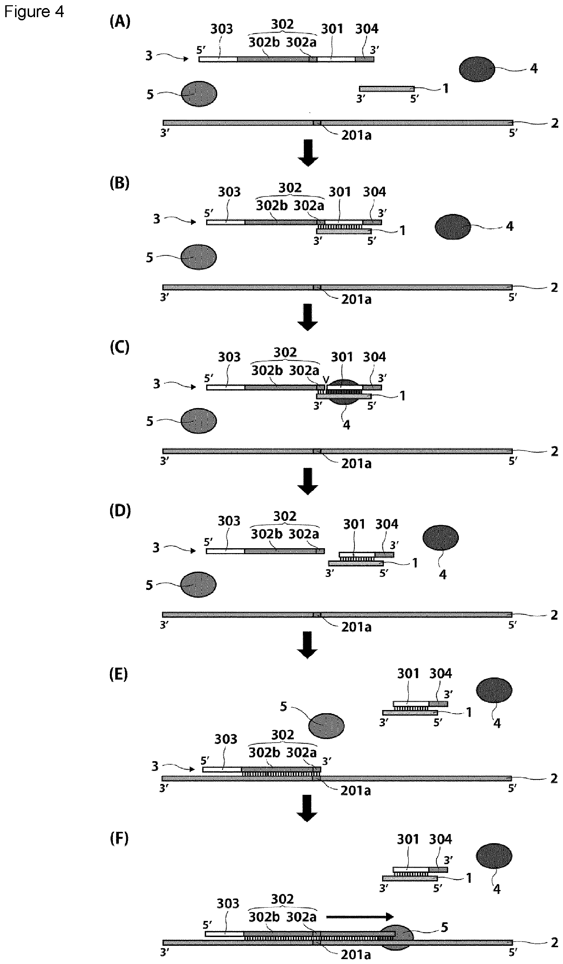

Fig. 4 is a schematic diagram showing a fourth embodiment of the method for nucleic acid detection of the present invention. Fig. 4 (A) shows a state in which a test sample containing a detection target nucleic acid and a reaction solution are mixed. Fig. 4 (B) shows a state in which the detection target nucleic acid and a primer for nucleic acid detection are hybridized to form a DNA-RNA complex. Fig. 4 (C) shows a state in which the second polynucleotide segment of the DNA-RNA complex is cleaved by a nuclease. Fig. 4 (D) shows a state in which the first polynucleotide segment has been removed from the primer for nucleic acid detection. Fig. 4 (E) shows a state in which an activated primer for nucleic acid detection is hybridized to a template nucleic acid. Fig. 4 (F) shows a state in which the template nucleic acid is amplified by a polymerase.
Fig. 5 is a drawing showing the sequence and the like of each primer used in an experiment for detecting a short DNA.
Fig. 6 is a drawing showing conditions of an experimental process for detecting a short DNA.
Fig. 7 is a drawing showing results of measuring the time required to confirm amplification by a LAMP method when detecting a short DNA.
Fig. 8 is a schematic diagram showing an outline of a nucleic acid amplification reaction of a LAMP method.
Fig. 9 is a schematic diagram showing an outline of a nucleic acid amplification reaction of a LAMP method.

### Description of Embodiments

### 1. Method for Nucleic Acid Detection

### 1-1. Overview of Method for Nucleic Acid Detection of Present Invention

A method for nucleic acid detection of a first invention of the present invention is characterized by using at least the following reagents A) to D).
A) A template nucleic acid which is amplified and serves as an indicator of detection
B) A primer for nucleic acid detection including a first polynucleotide segment having a base sequence complementary to at least a portion of the detection target nucleic acid and a second polynucleotide segment having a base sequence complementary to a portion of the template nucleic acid, wherein
   when the detection target nucleic acid is RNA, at least the 5' end of the first polynucleotide segment is DNA, and when the detection target nucleic acid is DNA, at least the 5' end of the first polynucleotide segment is RNA, and
   the second polynucleotide segment is linked to the 5' side of the first polynucleotide segment
C) A nuclease that degrades a DNA-RNA complex
D) A polymerase

In the method for nucleic acid detection of the present invention, by using the above-described reagents A) to D), when the detection target nucleic acid is contained in a test sample, the following reactions i) to iii) occur, and the presence of a detection target nucleic acid can be detected using amplification of a template nucleic acid as an index.
i) contacting a test sample containing the detection target nucleic acid with the primer for nucleic acid detection B) to hybridize the detection target nucleic acid and the primer for nucleic acid detection to form a DNA-RNA complex,
ii) removing the first polynucleotide segment from the primer for nucleic acid detection by degrading at least a portion of the DNA-RNA complex with the nuclease C), and
iii) hybridizing the primer for nucleic acid detection to the template nucleic acid A) and amplifying the template nucleic acid with the polymerase D).

Hereinafter, the reactions i) to iii) will be described with reference to the drawings.

Fig. 1 is a drawing schematically showing a first embodiment which is an example of the method for nucleic acid detection of the present invention.

Fig. 1 (A) shows a state in which a test sample containing a detection target nucleic acid and a reaction solution are mixed, and a detection target nucleic acid 1, a template nucleic acid 2, a primer for nucleic acid detection 3, a nuclease 4, and a polymerase 5 exist in the mixed reaction solution.

The template nucleic acid 2, the nuclease 4, and the polymerase 5 may be mixed from the beginning as in the first embodiment shown in Fig. 1, or may be added later depending on a reaction stage.

As shown in Fig. 1 (A), the primer for nucleic acid detection 3 includes a first polynucleotide segment 301 having a base sequence complementary to at least a part of the detection target nucleic acid 1 and a second polynucleotide segment 302 having a base sequence complementary to a part of the template nucleic acid 2. The second polynucleotide segment 302 is linked to the 5' side of the first polynucleotide segment 301.

The detection target nucleic acid 1 is RNA or DNA, when the detection target nucleic acid 1 is RNA, the first polynucleotide segment 301 is designed to be DNA, and when the detection target nucleic acid 1 is DNA, the first polynucleotide segment 302 is designed to be RNA.

Fig. 1 (B) shows a state in which a detection target nucleic acid and a first polynucleotide segment are hybridized to form a DNA-RNA complex.

As shown in Fig. 1 (B), the first polynucleotide segment 301 of the primer for nucleic acid detection 3 has a base sequence complementary to at least a portion of the detection target nucleic acid 1, and therefore, the first polynucleotide segment 301 and the detection target nucleic acid 1 are hybridized to form a DNA-RNA complex.

Fig. 1 (C) shows a state in which the first polynucleotide segment of the DNA-RNA complex is degraded by nuclease.

As shown in Fig. 1 (C), when a DNA-RNA complex is formed, a nuclease 4 recognizes and binds to the complex.

When the detection target nucleic acid 1 is RNA, the first polynucleotide segment is DNA, and therefore, a deoxyribonuclease (DNase) that degrades DNA in DNA-RNA complexes is used as the nuclease 4, thereby degrading the DNA of the first polynucleotide segment 301. When the detection target nucleic acid 1 is DNA, the first polynucleotide segment is RNA, and therefore, a ribonuclease (RNase) that degrades RNA in DNA-RNA complexes is used as the nuclease 4, thereby degrading the RNA of the first polynucleotide segment 301.

As the nuclease used in the present invention, an endonuclease that degrades nucleic acid inside the nucleic acid sequence can be used, and an exonuclease that degrades nucleic acid from the outside in the nucleic acid sequence can also be used.

In the first embodiment, an endonuclease is used as the nuclease 4, and degradation is started from a linking portion between the first polynucleotide segment 301 and the second polynucleotide segment 302, whereby the first polynucleotide segment 301 and the second polynucleotide segment 302 are separated. In this way, the first polynucleotide segment 301 is removed from the primer for nucleic acid detection 3 by the nuclease 4, and the primer for nucleic acid detection 3 is activated.

In the present invention, when an exonuclease is used as a nuclease, it is preferable to use an exonuclease that degrades nucleic acid from the 3' end, and as a result, the first polynucleotide segment is degraded from the 3' end, and the first polynucleotide segment is completely degraded, thereby activating the primer for nucleic acid detection.

In order to remove the first polynucleotide quickly and reliably, it is preferable to use an endonuclease as a nuclease.

Fig. 1 (D) shows a state in which the activated primer for nucleic acid detection is hybridized to a template nucleic acid, and Fig. 1 (E) shows a state in which the template nucleic acid is amplified by a polymerase.

Since the first polynucleotide segment 301 is removed, the primer for nucleic acid detection 3 is activated as a primer for amplifying the template nucleic acid 2. Specifically, as shown in Fig. 1 (D), when the primer for nucleic acid detection 3 is hybridized to the template nucleic acid 2, bases on the 3' end side of the primer for nucleic acid detection 3 forms base pairs with the template nucleic acid 2, and as a result, as shown in Fig. 1 (E), an amplification reaction by the polymerase 5 becomes possible.

On the other hand, in the method for nucleic acid detection of the present invention, when no detection target nucleic acid is present, the first polynucleotide segment is not removed from the primer for nucleic acid detection, and even when the primer for nucleic acid detection hybridizes to the template nucleic acid, bases on the 3' end side of the primer for nucleic acid detection does not form base pairs with the template nucleic acid, and therefore, a template nucleic acid amplification reaction cannot be performed normally.

Therefore, according to the method for nucleic acid detection of the present invention, a normal amplification reaction of a template nucleic acid occurs only when a detection target nucleic acid is present.

As shown in Fig. 1 (D), a primer for nucleic acid detection used in the present invention may hybridize to the template nucleic acid 2 after the first polynucleotide segment 301 is removed, or after the primer hybridizes to the template nucleic acid, the first polynucleotide segment may be removed by a nuclease.

In amplifying the template nucleic acid 2, a primer that hybridizes to a region of the template nucleic acid different from the primer for nucleic acid detection 3 can be designed, and the template nucleic acid 2 can be amplified by a PCR reaction using the two primers.

In amplifying the template nucleic acid 2, a LAMP method can also be used. The LAMP method is a nucleic acid amplification method developed by the present applicant (Japanese Patent No. 3313358 and the like), and is a method that enables an isothermal nucleic acid amplification reaction.

In a LAMP method, at least four types of primers designed based on the base sequences of six regions on a template nucleic acid are used as primers, and the primers are respectively called inner primer F (FIP), inner primer B (BIP), outer primer F (F3), and outer primer B (B3). In addition, loop primer F (LF) and loop primer B (LB) may also be used.

When the primer for nucleic acid detection of the present invention is designed as an inner primer, a third polynucleotide segment is provided on the 5' side of the second polynucleotide segment of the primer for nucleic acid detection. The base sequence of the third polynucleotide segment is designed to include the same base sequence as a part of the base sequence of a region on the 5' side from a region complementary to the second polynucleotide segment in the template nucleic acid.

On the other hand, when the primer for nucleic acid detection of the present invention is also designed as an outer primer, it is not necessary to provide the third polynucleotide segment.

An amplified template nucleic acid can be detected using optical means, for example, although not limited thereto, by reacting with an intercalator to be stained, or by using a primer labeled with a fluorescent dye or the like during nucleic acid amplification.

According to the method for nucleic acid detection of the present invention, an amplification reaction of a template nucleic acid occurs only when a detection target nucleic acid is present, and therefore a detection target nucleic acid can be detected using amplification of a template nucleic acid as an index.

Since the method for nucleic acid detection of the present invention, which is a method utilizing nucleic acid amplification, does not directly amplify a detection target nucleic acid, even a short nucleic acid having a size of from 10 to 100 bases that is difficult to directly amplify can be suitably detected.

### 1-2. Detailed Description of Method for Nucleic Acid Detection of Present Invention

In the present invention, a "detection target nucleic acid" to be detected is DNA (deoxyribonucleic acid) or RNA (ribonucleic acid), or may be DNA or RNA partially containing an artificial nucleic acid, or DNA or RNA bound to another substance as long as the target can form a DNA-RNA complex and can be degraded by a nuclease.

For the purpose of clinical diagnosis, an artificially extracted natural DNA or RNA present in a living body is preferably used as the "detection target nucleic acid".

Examples of natural DNA or RNA present in a living body include, but are not limited to, genomic DNA, mitochondrial DNA (mtDNA), messenger RNA (mRNA), transfer RNA (tRNA), ribosomal RNA (rRNA), small nuclear RNA (snRNA), small nucleolar RNA (snoRNA), microRNA (miRNA), and small interfering RNA (siRNA).

The length of a detection target nucleic acid is not particularly limited as long as the target can be recognized by a nuclease when a DNA-RNA complex is formed, and the length is usually from 5 to 5,000 bases.

Since the method for nucleic acid detection of the present invention, which is a method utilizing nucleic acid amplification, does not directly amplify a detection target nucleic acid, even a short nucleic acid having a size of from 10 to 100 bases that is difficult to directly amplify can be suitably detected.

A detection target nucleic acid is more preferably from 20 to 50 bases, and miRNA can also be suitably detected.

As the "template nucleic acid" used herein, DNA, RNA, or an artificial nucleic acid that is an analog thereof can be used, and DNA is preferably used from the viewpoint of easy amplification. As the "template nucleic acid", a stable double-stranded nucleic acid can be used, or may be a single-stranded nucleic acid.

In the present invention, as the artificial nucleic acid, for example, an artificial nucleic acid with a modified nucleotide skeleton composed of sugar and phosphate, or an artificial nucleic acid with a modified base can be used. Examples of the artificial nucleic acid with a modified nucleotide skeleton include, but are not limited to, peptide nucleic acid (PNA) with a peptide chain as a skeleton, locked nucleic acid (LNA) with a sugar cross-linked structure, and glycol nucleic acid (GNA) with glycol linked by phosphodiester bonds as a skeleton. Examples of the artificial nucleic acid with a modified base include, but are not limited to, an artificial nucleic acid using 2-amino-6-dimethylaminopurine, pyridone-2-one, 2-amino-6-(2-thienyl) purine, pyrrolopyridine, methyl isocarbosteryl, azaadenine, azaguanine, or the like as a base.

The "template nucleic acid" used herein does not necessarily need to be designed separately according to the sequence of a detection target nucleic acid, and one template nucleic acid can be commonly used in detection of a variety of detection target nucleic acids. Here, the "template nucleic acid" is preferably one having low homology to any of a variety of detection target nucleic acids, and this can prevent the template nucleic acid from hybridizing with a first polynucleotide segment of a primer for nucleic acid detection.

More preferably, a template nucleic acid having a non-natural artificial base sequence is used as the "template nucleic acid". Here, the "non-natural artificial base sequence" means not a base sequence of nucleic acid derived from an organism but an artificially prepared base sequence, and those with low homology to any of the nucleotide sequences of biological nucleic acids registered in the gene database is preferable. Using a non-natural artificial base sequence can prevent a template nucleic acid from hybridizing with a first polynucleotide segment of a primer for nucleic acid detection or a biologically-derived nucleic acid present in a test sample, and can further reduce contamination risk.

The "primer for nucleic acid detection" used herein includes a first polynucleotide segment having a base sequence complementary to at least a portion of the detection target nucleic acid, and a second polynucleotide segment having a base sequence complementary to a portion of a template nucleic acid.

Here, the first polynucleotide segment may be one having a base sequence complementary to all base sequences of a detection target nucleic acid or one having a base sequence complementary to only a part of a detection target nucleic acid.

"Having a complementary base sequence" herein means having a base sequence capable of forming base pairs that can be bonded to each other by hydrogen bonding as in an A (adenine) and T (thymine) base pair, a G (guanine) and C (cytosine) base pair, an A (adenine) and U (uracil) base pair, or a base pair of 2-amino-6-dimethylaminopurine and pyridon-2-one, and also may include having a mismatched base that does not form a base pair that can be bound by hydrogen bonding in part. It is noted that a boundary portion between a first polynucleotide segment and a second polynucleotide segment preferably does not have a mismatch, and in the first polynucleotide segment and the second polynucleotide segment, the percentage of mismatches is preferably 20% or less, and more preferably 5% or less.

In the primer for nucleic acid detection used in the present invention, a second polynucleotide segment is linked to the 5' side of a first polynucleotide segment. Here, an intervening sequence of about one base that is complementary to neither a detection target nucleic acid nor a template nucleic acid can be provided between the first polynucleotide segment and the second polynucleotide segment in some cases. Such an intervening sequence can be removed together under some conditions when the first polynucleotide segment is removed from a primer for nucleic acid detection, and in that case, the primer for nucleic acid detection can be activated.

The primer for nucleic acid detection used in the present invention may have a nucleic acid segment other than the first polynucleotide segment and the second polynucleotide segment. For example, a third polynucleotide segment that is not complementary to the base sequence of a template nucleic acid can be linked to the 5' side of a second polynucleotide segment. A fourth polynucleotide segment that is not complementary to the base sequence of a detection target nucleic acid can be linked to the 3' side of a first polynucleotide segment.

A third polynucleotide segment can also have a specific function. For example, although not limited thereto, by designing the third polynucleotide segment in such a manner to include the same base sequence as a part of the base sequence of a region on the 5' side of a region complementary to a second polynucleotide segment in a template nucleic acid, a primer for nucleic acid detection can be used as an inner primer of a LAMP method.

A fourth polynucleotide segment can also have a specific function. For example, although not limited thereto, by designing the fourth polynucleotide segment to have a non-natural artificial base sequence and not to hybridize with a template nucleic acid, even when the first polynucleotide segment has hybridized with a nucleic acid other than a detection target nucleic acid in a test sample, or even when a detection target nucleic acid has high homology with a template nucleic acid and the first polynucleotide segment has hybridized to a template nucleic acid, the fourth polynucleotide segment does not hybridize with the nucleic acid or template nucleic acid in the test sample, and therefore, an unintended amplification reaction can be prevented.

Although in a primer for nucleic acid detection, a first polynucleotide segment must be individually designed according to the sequence of a detection target nucleic acid, second to fourth polynucleotide segments do not necessarily have to be designed according to the sequence of a detection target nucleic acid, and a common sequence can be used in detection of a variety of detection target nucleic acids. Consequently, in the method for nucleic acid detection of the present invention, when designing a primer for nucleic acid detection, at least only a first polynucleotide segment needs to be designed according to the sequence of a detection target nucleic acid, and such a primer can be designed easily.

Fig. 2 is a drawing schematically showing a second embodiment which is another example of the method for nucleic acid detection of the present invention.

Fig. 2 (A) shows a state in which a test sample containing a detection target nucleic acid and a reaction solution are mixed, and the primer for nucleic acid detection 3 includes a third polynucleotide segment 303 and a fourth polynucleotide segment 304 in addition to the first polynucleotide segment 301 and the second polynucleotide segment 302. The first polynucleotide segment 301 has a base sequence complementary to only a portion of the detection target nucleic acid 1.

Fig. 2 (B) shows a state in which a detection target nucleic acid and a first polynucleotide segment are hybridized to form a DNA-RNA complex, and since the first polynucleotide segment 301 has a base sequence complementary to only a portion of the detection target nucleic acid 1, the detection target nucleic acid 1 includes a portion that does not form a DNA-RNA complex.

Fig. 2 (C) shows a state in which a DNA-RNA complex is degraded by a nuclease, and degradation is started from a linking portion between the first polynucleotide segment 301 and the second polynucleotide segment 302, whereby the first polynucleotide segment 301 and the second polynucleotide segment 302 are separated.

In this way, even when the first polynucleotide segment 301 has a base sequence complementary to only a portion of the detection target nucleic acid 1, the first polynucleotide segment 301 can be removed from the primer for nucleic acid detection 3 to activate the primer for nucleic acid detection 3.

Fig. 2 (D) shows a state in which the activated primer for nucleic acid detection is hybridized to a template nucleic acid, and Fig. 2 (E) shows a state in which the template nucleic acid is amplified by a polymerase.

The primer for nucleic acid detection 3 includes a third polynucleotide segment 303, and the base sequence thereof is the same as a portion of the base sequence of a region on the 5' side of a region of the template nucleic acid 2 to which the second polynucleotide segment 302 hybridizes. As a result, the primer for nucleic acid detection 3 can be used as an inner primer of a LAMP method, and furthermore, another inner primer and two outer primers can be designed and the template nucleic acid 2 can be isothermally amplified by the LAMP method using a polymerase 5.

In the primer for nucleic acid detection used in the present invention, the first polynucleotide segment is a nucleic acid with DNA or RNA at least on the 5' end, but is not limited to a nucleic acid consisting only of DNA and/or RNA, and may be an artificial nucleic acid in part as long as the segment is one that can hybridize with a detection target nucleic acid to form a DNA-RNA complex and can be degraded by a nuclease, or may be a nucleic acid bound to another substance such as a label.

As long as the first polynucleotide segment includes a base sequence complementary to at least a portion of a detection target nucleic acid, the base length and sequence thereof are not particularly limited, and it is preferable to design the base length and sequence to obtain a Tm value suitable for hybridization. The base length of the first polynucleotide segment is usually from 5 to 5,000 bases, more preferably 10 to 100 bases, and further preferably 20 to 50 bases.

The first polynucleotide segment may include a portion complementary to a detection target nucleic acid and complementary to a template nucleic acid. The second polynucleotide segment may include a portion complementary to a detection target nucleic acid and complementary to a template nucleic acid.

In the present invention, when a detection target nucleic acid is RNA, at least the 5' end of a first polynucleotide segment is DNA. In this case, from the viewpoint of ease of production of a primer for nucleic acid detection, the first polynucleotide segment is preferably all DNA.

When the detection target nucleic acid is DNA, at least the 5' end of the first polynucleotide segment is RNA. In this case, the first polynucleotide segment may be all RNA, or only the 5' end of the first polynucleotide segment may be RNA.

By designing a primer for nucleic acid detection in such a manner that a DNA-RNA complex is formed when a detection target nucleic acid and a primer for nucleic acid detection are hybridized, and performing nuclease degradation on at least a portion of the DNA-RNA complex, the primer for nucleic acid detection can be activated.

Fig. 3 is a drawing schematically showing a third embodiment which is another example of the method for nucleic acid detection of the present invention.

Fig. 3 (A) shows a state in which a test sample containing a detection target nucleic acid and a reaction solution are mixed, and a primer for nucleic acid detection 3 which is one of reagents includes a first polynucleotide segment 301 and a second polynucleotide segment 302. In the third embodiment, a detection target nucleic acid 1 is DNA in its full length. The first polynucleotide segment 301 of the primer for nucleic acid detection 3 has a base sequence complementary to a portion of the detection target nucleic acid 1, and the 5' end is RNA and the other portion is DNA.

In the third embodiment, the second polynucleotide segment 302 is composed of a DNA having a base sequence complementary to a portion of a template nucleic acid 2, and is designed to be divided into a 3' side segment 302a and a 5' side segment 302b. The 3' side segment 302a is designed not only to be complementary to a portion of the template nucleic acid 2 but also to a portion of the detection target nucleic acid 1. The same base sequence (common base sequence) 201a as a portion of the detection target nucleic acid 1 is inserted in the template nucleic acid 2, and thus the template nucleic acid 2 can be designed in such a manner that 302a is not only complementary to a portion of the template nucleic acid 2 but also complementary to a portion of the detection target nucleic acid 1.

Fig. 3 (B) shows a state in which a detection target nucleic acid and a primer for nucleic acid detection are hybridized to form a DNA-RNA complex.

As shown in Fig. 3 (B), a first polynucleotide segment 301 and a portion 302a on the 3' side of a second polynucleotide segment hybridize with a detection target nucleic acid 1. Here, since only the 5' end of the first polynucleotide segment 301 is RNA, a DNA-RNA complex is formed in this portion.

Fig. 3 (C) shows a state in which the first polynucleotide segment of the DNA-RNA complex is degraded by a nuclease.

As shown in Fig. 3 (C), when a DNA-RNA complex is formed, a nuclease 4 recognizes this complex and binds thereto, and degrades RNA in the DNA-RNA complex. In the third embodiment, RNase H is used as a nuclease, and even when the DNA-RNA complex is only one base pair, the nuclease can recognize and decompose the complex. Although this is a degradation of only one base, the boundary between the first polynucleotide segment 301 and the second polynucleotide portion 302 is cleaved.

Fig. 3 (D) shows a state in which the first polynucleotide segment has been removed from the primer for nucleic acid detection.

In the third embodiment, as shown in Fig. 3 (C), although the boundary between the first polynucleotide segment 301 and the second polynucleotide segment 302 is cleaved, a segment 302a on the 3' side of the second polynucleotide segment is hybridized with the detection target nucleic acid 1, and therefore, the first polynucleotide segment 301 is not cut off from the primer for nucleic acid detection 3 as it is. However, since base pairing and separation are in dynamic equilibrium, as shown in Fig. 3 (D), the first polynucleotide segment 301 can be separated from the primer for nucleic acid detection 3. Alternatively, by heating a reaction solution, separation of the first polynucleotide segment can be promoted.

Fig. 3 (E) shows a state in which the primer for nucleic acid detection is hybridized to the template nucleic acid, and Fig. 3 (F) shows a state in which the template nucleic acid is amplified by a polymerase.

As shown in Fig. 3 (E), the primer for nucleic acid detection 3 activated by removal of the first polynucleotide segment 301 hybridizes to the template nucleic acid 2. Since the segment 302a on the 3' side of the second polynucleotide segment is designed to be complementary to a portion of the detection target nucleic acid 1 and also complementary to a common base sequence 201a of the template nucleic acid 2, a base at the 3' end of the primer for nucleic acid detection 3 forms base pairs with the template nucleic acid 2. For this reason, as shown in Fig. 3 (F), an amplification reaction by a polymerase 5 becomes possible.

In the primer for nucleic acid detection used in the present invention, a second polynucleotide segment may be DNA, RNA, or an artificial nucleic acid that is an analog thereof, and is preferably DNA in view of ease of nucleic acid amplification.

As long as the second polynucleotide segment includes a base sequence complementary to a portion of a template nucleic acid, the base length and sequence thereof are not particularly limited, and it is preferable to design the base length and sequence to obtain a Tm value suitable for hybridization.

As in the example of the third embodiment, the second polynucleotide segment may be such that the 3' side portion is complementary to a template nucleic acid and is also complementary to a detection target nucleic acid.

The primer for nucleic acid detection used in the present invention can be chemically synthesized, for example, although not limited thereto, by using a solid phase synthesis method or the like. A primer for nucleic acid detection, a portion of which is RNA and the other portion of which is DNA, can also be chemically synthesized.

The primer for nucleic acid detection used in the present invention preferably includes a modification at the 3' end to stop an amplification reaction. By providing such modification, it is possible to prevent a primer for nucleic acid detection from which a first polynucleotide segment has not been removed from hybridizing with a template nucleic acid or a nucleic acid other than a detection target nucleic acid in a test sample to cause a nucleic acid amplification reaction.

For example, although not limited thereto, by performing a modification that replaces an OH group at the position 3' of ribose at the end of a polynucleotide with another substance such as a phosphate group as a modification that stops an amplification reaction, an extension reaction by a polymerase can be stopped.

Alternatively, as described above, by linking a fourth polynucleotide segment to the 3' end of a first polynucleotide segment to design the fourth polynucleotide segment to include a non-natural artificial base sequence and not to hybridize with a template nucleic acid, when the first polynucleotide segment has hybridized with a nucleic acid other than a detection target nucleic acid in a test sample, or even when the first polynucleotide segment has hybridized to a template nucleic acid, it is possible to prevent an unintended nucleic acid amplification reaction from occurring since the fourth polynucleotide segment does not hybridize with a nucleic acid or a template nucleic acid in a test sample.

As the "nuclease" used herein, when a detection target nucleic acid is RNA, a deoxyribonuclease (DNase) that degrades DNA in a DNA-RNA complex is used. When a detection target nucleic acid is DNA, a ribonuclease (RNase) that degrades RNA in a DNA-RNA complex is used as the nuclease.

As the nuclease used herein, an endonuclease that degrades a nucleic acid within the nucleic acid sequence can be used, and an exonuclease that degrades a nucleic acid from the outside of the nucleic acid sequence can also be used.

In order to remove a first polynucleotide quickly and reliably, it is preferable to use an endonuclease as the nuclease. When using an exonuclease, it is preferable to use an exonuclease which decomposes a nucleic acid from the 3' end.

Examples of the deoxyribonuclease (DNase) include, but are not limited to, DNase H (International Publication No. 2014/190181, Mol Cell., 2015, Oct 15, 60 (2), pp.242-255), which is an endonuclease that degrades DNA in DNA-RNA complexes. Examples of ribonuclease (RNase) include, but are not limited to, RNase H, which is an endoribonuclease that degrades RNA in DNA-RNA complexes.

The DNase H is a protein called NmeCas9, and is a kind of Cas9 protein that functions as a nuclease in a CRISPR-Cas9 system, which is an adaptive immune system possessed by bacteria or archaea. A CRISPR-Cas9 system has attracted attention as being applicable to genome editing technology, and a Cas9 protein commonly used in genome editing technology is a SpyCas9 derived from *Streptococcus pyogenes.* On the other hand, NmeCas9 is a Cas9 protein derived from *Neisseria meningitidis*, and has different characteristics from SpyCas9. In the presence of crRNA and tracrRNA that function as a guide strand, SpyCas9 from *Streptococcus pyogenes* binds to double-stranded DNA having a sequence complementary to a part of crRNA and a PAM sequence to cleave the double-stranded DNA. On the other hand, NmeCas9 derived from *Neisseria meningitidis* does not require a tracrRNA or PAM sequence, and when crRNA is present, NmeCas9 binds to a single-stranded DNA having a sequence complementary to a portion of the crRNA and cleaves the single-stranded DNA. Furthermore, in an RNA-DNA complex of at least 17 bases or more, NmeCas9 can cleave single-stranded DNA regardless of the sequence. Since NmeCas9 cleaves the DNA strand in a RNA-DNA complex, NmeCas9 is said to have DNase H activity from the comparison with RNase H, which degrades the RNA strand in a RNA-DNA complex (Mol Cell., 2015, Oct 15, 60 (2), pp.242-255).

Fig. 4 is a drawing schematically showing a fourth embodiment as an example of a method of using NmeCas9 as a nuclease in the method for nucleic acid detection of the present invention.

Fig. 4 (A) shows a state in which a test sample containing a detection target nucleic acid and a reaction solution are mixed. In Fig. 4 (A), a primer for nucleic acid detection 3 which is one of reagents includes a first polynucleotide segment 301, a second polynucleotide segment 302, a third polynucleotide segment 303, a fourth polynucleotide segment 304. In the fourth embodiment, a detection target nucleic acid 1 is RNA in its full length. The first polynucleotide segment 301 of the primer for nucleic acid detection 3 has a base sequence complementary to at least a portion of the detection target nucleic acid 1.

In the fourth embodiment, the second polynucleotide segment 302 is composed of a DNA having a base sequence complementary to a portion of a template nucleic acid 2, and is designed to be divided into a 3' side segment 302a and a 5' side segment 302b. The 3' side segment 302a is designed not only to be complementary to a portion of the template nucleic acid 2 but also to a portion of the detection target nucleic acid 1. When NmeCas9 is used, it is necessary to provide such a segment 302a with one base or more, and more preferably from 2 to 20 bases. The same base sequence (common base sequence) 201a as a portion of the detection target nucleic acid 1 is inserted in the template nucleic acid 2, and thus the template nucleic acid 2 can be designed in such a manner that 302a is not only complementary to a portion of the template nucleic acid 2 but also complementary to a portion of the detection target nucleic acid 1.

Fig. 4 (B) shows a state in which a detection target nucleic acid and a primer for nucleic acid detection are hybridized to form a DNA-RNA complex.

As shown in Fig. 4 (B), a first polynucleotide segment 301 and a portion 302a on the 3' side of a second polynucleotide segment continuously hybridize with a detection target nucleic acid 1 to form a DNA-RNA complex.

Fig. 4 (C) shows a state in which the second polynucleotide segment of the DNA-RNA complex is cleaved by a nuclease.

As shown in Fig. 4 (C), when a DNA-RNA complex is formed, a nuclease 4 (NmeCas9) recognizes this complex and binds thereto, and cleaves a DNA strand at a distance of 2 to 5 bases from the 5' end of the DNA strand in the DNA-RNA complex. The site to be cleaved can be slightly shifted by adding tracrRNA or the like. The site to be cleaved by the nuclease 4 is in the second polynucleotide segment, and may be the boundary between the first polynucleotide segment and the second polynucleotide segment.

Fig. 4 (D) shows a state in which the first polynucleotide segment has been removed from the primer for nucleic acid detection.

In the fourth embodiment, as shown in Fig. 4 (C), although the primer for nucleic acid detection 3 is cleaved, a portion of a segment 302a on the 3' side of the second polynucleotide segment is hybridized with the detection target nucleic acid 1, and therefore, the first polynucleotide segment 301 is not cut off from the primer for nucleic acid detection 3 as it is. However, since base pairing and separation are in dynamic equilibrium, as shown in Fig. 4 (D), the first polynucleotide segment 301 can be separated from the primer for nucleic acid detection 3. Alternatively, by heating a reaction solution, separation of the first polynucleotide segment can be promoted.

Fig. 4 (E) shows a state in which the primer for nucleic acid detection is hybridized to the template nucleic acid, and Fig. 4 (F) shows a state in which the template nucleic acid is amplified by a polymerase.

As shown in Fig. 4 (E), the primer for nucleic acid detection 3 activated by removal of the first polynucleotide segment 301 hybridizes to the template nucleic acid 2. Since the segment 302a on the 3' side of the second polynucleotide segment is designed to be complementary to a portion of the detection target nucleic acid 1 and also complementary to a common base sequence 201a of the template nucleic acid 2, a base at the 3' end of the primer for nucleic acid detection 3 forms base pairs with the template nucleic acid 2. For this reason, as shown in Fig. 4 (F), an amplification reaction by a polymerase 5 becomes possible.

As the "polymerase" used herein, a polymerase that synthesizes DNA using DNA as a template (DNA-dependent DNA polymerase) or a polymerase that synthesizes DNA using RNA as a template (RNA-dependent DNA polymerase) can be used, and it is preferable to use a DNA-dependent DNA polymerase in view of ease of nucleic acid amplification.

The step i) of the method for nucleic acid detection of the present invention is a step of contacting a test sample containing the detection target nucleic acid with a primer for nucleic acid detection to hybridize the detection target nucleic acid and the primer for nucleic acid detection to form a DNA-RNA complex.

Here, "hybridization" means that a detection target nucleic acid and a first polynucleotide segment form a complex by binding of a base of the detection target nucleic acid and a base of the first polynucleotide segment through hydrogen bonding.

When a detection target nucleic acid is RNA, at least the 5' end of a first polynucleotide segment is DNA, and when a detection target nucleic acid is DNA, at least the 5' end of a first polynucleotide segment is RNA, and therefore, when the detection target nucleic acid and the first polynucleotide segment are hybridized, a DNA-RNA complex is formed at least at the 5' end of the first polynucleotide segment.

Conditions for bringing a test sample containing a detection target nucleic acid into contact with a primer for nucleic acid detection are not particularly limited as long as they can hybridize and form a DNA-RNA complex, and the reaction conditions are preferably such that only those with high homology can hybridize.

As such reaction conditions, conditions such as temperature, salt concentration, pH, and organic solvent to be added can be set in consideration of the base sequence of a detection target nucleic acid and the base sequence of a first polynucleotide segment.

The step ii) of the method for nucleic acid detection of the present invention is a step of removing a first polynucleotide segment from a primer for nucleic acid detection by degrading at least a portion of a DNA-RNA complex with a nuclease.

Here, "at least a portion of a DNA-RNA complex" may be a portion capable of removing a first polynucleotide segment from a primer for nucleic acid detection by degrading the portion with a nuclease. For example, when a first polynucleotide segment is degraded using an endonuclease, it is sufficient that at least a DNA-RNA complex at the 5' end of the first polynucleotide segment is degraded.

The "degradation" may be any degradation as long as at least a portion of covalent bonds of a nucleotide skeleton is at least degraded to cleave the nucleotide skeleton.

Reaction conditions for performing degradation with a nuclease are not particularly limited as long as the reaction conditions allow a first polynucleotide segment of a DNA-RNA complex to be degraded, and it is preferable to carry out a reaction using a solvent containing a necessary metal ion or the like at an optimum temperature, optimum salt concentration, and optimum pH according to the type of a nuclease to be used.

The step iii) of the method for nucleic acid detection of the present invention is a step of hybridizing a primer for nucleic acid detection to a template nucleic acid and amplifying a template nucleic acid with a polymerase.

Here, a reaction for amplifying a template nucleic acid is not particularly limited as long as the reaction is a nucleic acid amplification method that requires a primer, and although not limited thereto, for example, a PCR method, a LAMP method, or an SDA method can be used.

In a polymerase chain reaction (PCR) method, two primers that are complementary to a template nucleic acid and hybridize to a sense strand and an antisense strand of the template nucleic acid are designed and used. Then, 1) thermal denaturation by heating a double-stranded nucleic acid to single-stranded nucleic acids, 2) annealing to hybridize a primer to a template nucleic acid, and 3) an extension reaction in which a nucleic acid is synthesized by a polymerase using a template nucleic acid as a template starting from a primer are carried out, and by repeating the steps 1) to 3), a nucleic acid in a region sandwiched between the primers can be amplified.

When a PCR method is used in the method for nucleic acid detection of the present invention, the primer for nucleic acid detection of the present invention is used as one primer, and the other primer is designed and used based on the base sequence of a template nucleic acid.

In annealing in the PCR method, it is preferable to calculate Tm (melting temperature) based on the base sequence of a second polynucleotide segment of the primer for nucleic acid detection of the present invention or the base sequence of the other primer, and set the annealing temperature to a temperature lower than Tm.

An extension reaction is usually carried out at about from 67 to 77°C, and thermal denaturation is usually carried out at about from 90 to 98°C.

Since annealing, an extension reaction, and thermal denaturation are carried out at different temperatures, a thermal cycler is usually used as a device for quickly shifting these temperatures.

In a PCR method, a thermostable DNA polymerase is usually used, and in this case, a reaction buffer containing four types of substrates (dATP, dGTP, dCTP, and dTTP) and divalent magnesium ions and serving as a buffer solution that maintains an optimum pH is used.

A LAMP method is a nucleic acid amplification method (Japanese Patent No. 3313358 and the like) developed by the applicant of the present application, and is a method that enables an isothermal nucleic acid amplification reaction using a strand displacement polymerase.

In the LAMP method, an inner primer pair (FIP and BIP) and an outer primer pair (F3 and B3) are used, and in addition, a loop primer pair (LF and LB) can be used.

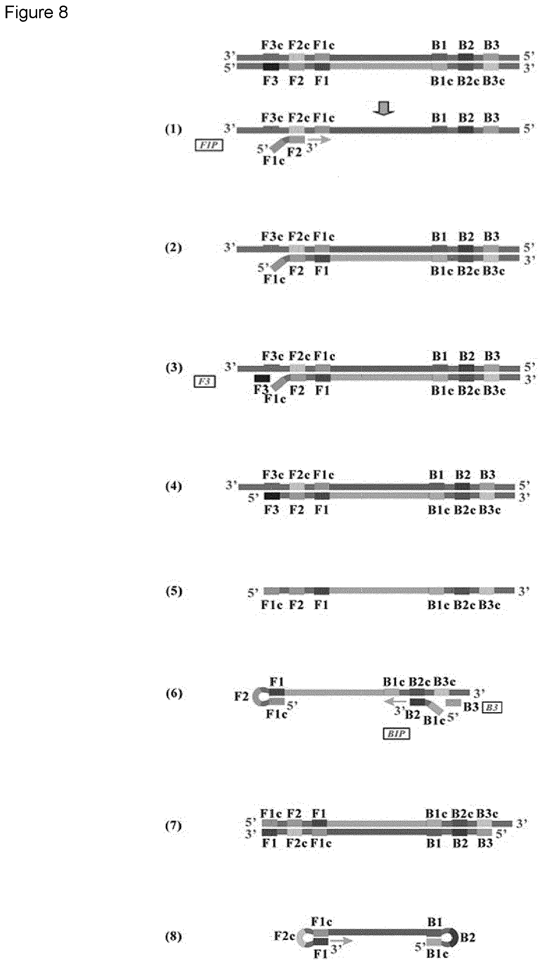

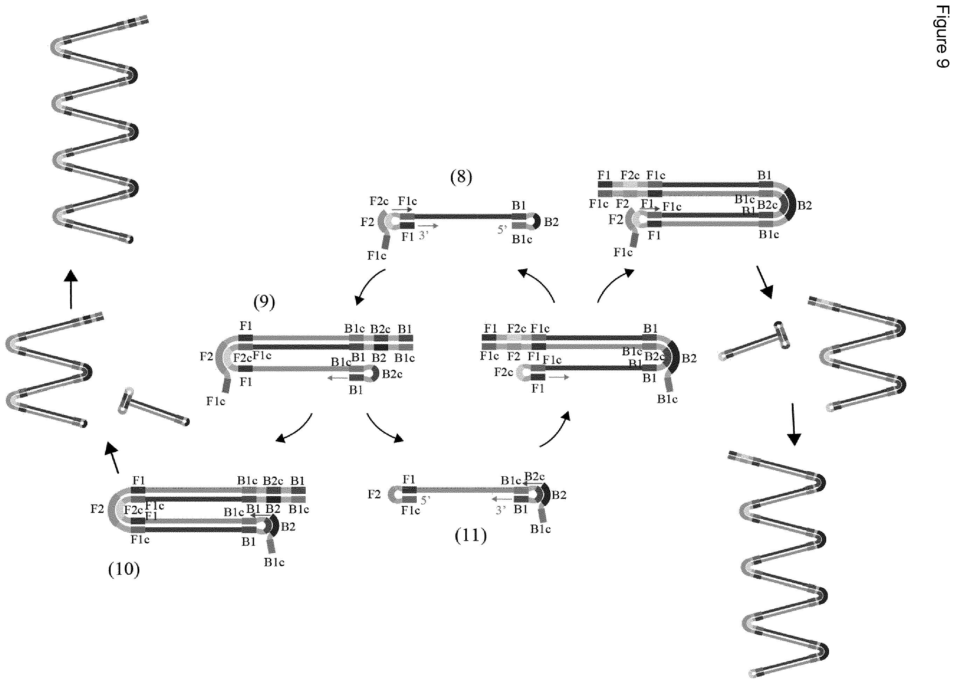

An outline of a nucleic acid amplification reaction of the LAMP method is schematically shown in Figs. 8 and 9.

As shown in Fig. 8 (1), when any sequence F1c present on one strand of a template nucleic acid and any sequence F2c present on the 3' side thereof are selected, an inner primer (FIP) is a primer having a sequence F2 complementary to F2c and a sequence F1c identical to F1c in this order from 3' to 5'.

As shown in Fig. 8 (6), when any sequence B1c present on the other strand of the template nucleic acid and any sequence B2c present on the 3' side thereof are selected, the inner primer (BIP) is a primer having a sequence B2c complementary to B2c and a sequence B1c identical to B1c in this order from 3' to 5'.

Since a template nucleic acid is in a state of dynamic equilibrium around 65°C even in a state of double-stranded nucleic acid, some primer anneals to a complementary portion of the template nucleic acid, a strand displacement polymerase is used, and then, one strand is peeled off by an extension reaction starting from the primer to make the template nucleic acid in a single-stranded state. For this reason, in a LAMP method, unlike a PCR method, it is not necessary to thermally denature double-stranded nucleic acids into single-stranded nucleic acids in advance. Then, as shown in Fig. 8 (1), an extension reaction can be initiated by annealing an inner primer to the single-stranded template nucleic acid. Consequently, as shown in Fig. 8 (2), a nucleic acid complementary to the template nucleic acid is synthesized.

In the method for nucleic acid detection of the present invention, when a primer for nucleic acid detection is used as an inner primer of a LAMP method, a second polynucleotide segment is designed to be F2 or B2, and further, a third polynucleotide segment is designed to have F1c or B1c.

As shown in Figs. 8 (3) and (6), an outer primer is a primer having a sequence of F3 or B3 complementary to any sequence F3c or B3c present outside (3' side) F2c or B2c complementary to an inner primer in a template nucleic acid.

As shown in Fig. 8 (3), an outer primer (F3) anneals outside an inner primer (FIP). Next, starting from the outer primer (F3), a nucleic acid complementary to the template nucleic acid is synthesized by a strand displacement polymerase, and a nucleic acid is synthesized while peeling off a nucleic acid previously synthesized starting from the inner primer (FIP). As a result, as shown in Fig. 8 (4), a double-stranded nucleic acid synthesized from the outer primer (F3) is generated.

A nucleic acid synthesized starting from the inner primer (FIP) is peeled off by nucleic acid synthesis starting from the outer primer (F3) to be a single strand, and includes complementary regions F1c and F1 on the 5' end side as shown in Fig. 8 (5). As a result, as shown in Fig. 8 (6), self-annealing occurs to form a loop.

As shown in Fig. 8 (6), an inner primer (BIP) anneals to a nucleic acid that forms a loop, and a complementary nucleic acid is synthesized starting therefrom. In this process, the loop is peeled and extended. Furthermore, the outer primer (B3) anneals outside the inner primer (BIP), using this as a starting point, a complementary nucleic acid is synthesized by a strand displacement polymerase, and a nucleic acid extends while peeling a nucleic acid previously synthesized from the inner primer (BIP). As a result, as shown in Fig. 8 (7), a double-stranded nucleic acid synthesized from the outer primer (B3) is generated.

In the method for nucleic acid detection of the present invention, a primer for nucleic acid detection can be used as an outer primer of a LAMP method, and in this case, a second polynucleotide segment is designed to be F3 or B3.

As shown in Fig. 8 (6), a nucleic acid synthesized starting from the inner primer (BIP) is peeled off by synthesis starting from the outer primer (B3) to be a single-stranded nucleic acid. As shown in Fig. 8 (8), since this nucleic acid includes complementary regions F1c and F1 on the 3' end side and complementary regions B1c and B1 on the 5' end side, a loop is formed at both ends thereof, resulting in a dumbbell-shaped nucleic acid.

A dumbbell-shaped nucleic acid has a structure serving as a starting point for causing exponential nucleic acid amplification.

As shown in Fig. 9 (8), First, nucleic acid synthesis is carried out using F1 at the 3' end of a dumbbell-shaped nucleic acid as a starting point, and the 5' end loop is peeled off and extended. Furthermore, since F2c of the loop on the 3' end side is single-stranded, the inner primer (FIP) or loop primer can anneal, nucleic acid synthesis is carried out starting from F2 of FIP, and a nucleic acid extends while a nucleic acid previously synthesized from F1 is peeled off.

Next, as shown in Fig. 9 (9), since a nucleic acid synthesized from F1, which is a single strand obtained by peeling by a nucleic acid synthesized from FIP, includes a complementary region on the 3' end side thereof, a loop is formed by self-annealing. Starting from B1 at the 3' end of this loop, synthesis of a nucleic acid using itself as a template is initiated. Then, extension proceeds while the nucleic acid synthesized from FIP is peeled off, and a structure shown in Fig. 9 (10) is obtained.

Through the above process, the nucleic acid synthesized from FIP is peeled off to be a single strand, and as shown in Fig. 9 (11), since complementary regions are contained at both ends thereof, a loop is formed at both ends, resulting in a dumbbell nucleic acid.

The structure of the dumbbell nucleic acid of Fig. 9 (11) is completely complementary to the structure of (8). For this reason, similarly to the dumbbell nucleic acid of (8), a nucleic acid is synthesized from B1 at the 3' end using itself as a template, then an inner primer (BIP) or a loop primer anneals to B2c of the loop on the 3' end side, and nucleic acid synthesis takes place from B2 of BIP to extend a nucleic acid while the nucleic acid previously synthesized from B1 is peeled off. As a result, a nucleic acid of (8) is produced again through the same processes as (8), (9), and (11).

In such a cycle of nucleic acid synthesis, nucleic acids such as (10) are synthesized one after another.

In the nucleic acid of (10), BIP or a loop primer anneals to a single-stranded B2c to synthesize a nucleic acid while a double-stranded segment is peeled off, then a nucleic acid with a loop is generated, and amplification products with a structure in which sequences complementary to each other are repeated on the same strand are generated in various sizes by repetition of a process by which a nucleic acid is synthesized from a loop.

In a LAMP method, nucleic acids are amplified exponentially by the amplification reaction as described above.

In a LAMP method, a strand displacement DNA polymerase is usually used, and in this case, a reaction buffer containing four types of substrates (dATP, dGTP, dCTP, and dTTP) and divalent magnesium ions and serving as a buffer solution that maintains an optimum pH is used.

The reaction temperature of a LAMP method is usually from 50 to 70°C, and preferably from 55 to 70°C. The reaction time is usually from 1 minute to 10 hours, and preferably from 5 minutes to 4 hours.

A standard displacement amplification (SDA) method is a method of amplifying a nucleic acid using a restriction enzyme and a strand displacement polymerase. In an SDA method, a restriction enzyme recognition sequence is inserted into a primer in advance, whereby a nick caused by a restriction enzyme treatment gives a starting point for complementary strand synthesis, and a nucleic acid extension reaction is carried out while the previously synthesized nucleic acid is peeled off therefrom, and therefore, a nucleic acid can be amplified isothermally without changing the temperature as in a PCR method.

In the method for nucleic acid detection of the present invention, the presence of a detection target nucleic acid can be detected using amplification of a template nucleic acid as an index.

Examples of a method for confirming amplification of a template nucleic acid include, but are not limited to, a method of optically detecting an amplified nucleic acid by reacting with an intercalator and staining and a method of optical detecting an amplified nucleic acid by preliminarily labeling a primer with a fluorescent dye and incorporating the fluorescent dye into the amplified nucleic acid. In order to confirm an amplified nucleic acid, an amplified nucleic acid having a large molecular weight may be separated by electrophoresis, or an amplified nucleic acid may be detected utilizing FRET (fluorescence resonance energy transfer) using a fluorescent dye.

Although not limited thereto, an intercalator that specifically binds to a double-stranded nucleic acid such as ethidium bromide, SYBR Green (registered trademark), or Pico Green (registered trademark) can be used.

### 2. Primer for Nucleic Acid Detection

The primer for nucleic acid detection of the second invention of the present invention is a reagent used for detection of a target nucleic acid, and a primer for nucleic acid detection described in the above 1. can be used.

A primer for nucleic acid detection may be a product dissolved in a buffer solution, or may be a product in a dried state.

A buffer solution may be any solution as long as the solution has a composition capable of stably dissolving a primer for nucleic acid detection. For example, although not limited thereto, a buffer in which Tris hydrochloric acid is dissolved, a TE buffer in which Tris hydrochloric acid and EDTA are dissolved, or the like can be used.

### 3. Kit for Nucleic Acid Detection

The kit for nucleic acid detection of the third invention of the present invention contains at least
A) a template nucleic acid that is amplified and serves as a detection index,
B) a primer for nucleic acid detection including a first polynucleotide segment having a base sequence complementary to at least a portion of a detection target nucleic acid and a second polynucleotide segment having a base sequence complementary to a portion of the template nucleic acid, wherein
   when the detection target nucleic acid is RNA, at least the 5' end of the first polynucleotide segment is DNA, and when the detection target nucleic acid is DNA, at least the 5' end of the first polynucleotide segment is RNA, and
   the second polynucleotide segment is linked to the 5' side of the first polynucleotide segment, and
C) a nuclease that degrades a DNA-RNA complex,
   as reagents.
   The kit for nucleic acid detection of the present invention can be used in combination with a commercially available polymerase, and it is preferable that the kit for nucleic acid detection of the present invention itself further contains
D) a polymerase
   as a reagent.
   As the reagents A) to D), the same reagents as described in the above-described 1. and 2. can be used.

The kit for nucleic acid detection of the present invention may contain instructions describing
i) contacting a test sample containing the detection target nucleic acid with the primer for nucleic acid detection to hybridize the detection target nucleic acid and the primer for nucleic acid detection to form a DNA-RNA complex,
ii) removing the first polynucleotide segment from the primer for nucleic acid detection by degrading at least a portion of the DNA-RNA complex with the nuclease, and
iii) hybridizing the primer for nucleic acid detection to the template nucleic acid and amplifying the template nucleic acid with the polymerase.

The present invention will be described in detail below with reference to Examples, but the present invention is not limited thereto.

### Example 1

### (Detection of short DNA)

A short DNA was detected using the method for nucleic acid detection of the present invention.

A DNA (miR-21) having a base sequence encoded by an MIR21 gene was used as a detection target nucleic acid, and an ARITA artificial gene was used as a template nucleic acid.

As a primer for nucleic acid detection, ART7-BIP-R including an RNA having a base sequence complementary to a portion of the miR-21 as a first polynucleotide segment, and a DNA having a base sequence complementary to a portion of the ARITA artificial gene as a second polynucleotide segment, and further including a third polynucleotide segment to serve as an inner primer for a LAMP method was designed.

ART7-BIP-D in which the first polynucleotide segment in ART7-BIP-R was replaced with a DNA sequence was designed as a control primer.

Further, ART7-BIP in which the first polynucleotide segment was removed in ART7-BIP-R was designed as a control primer.

Still further, ART7-FIP was designed as another inner primer in an LAMP method, ART7-F3 and ART7-B3 were designed as outer primers, and ART7-LF and ART7-LB were designed as loop primers.

As a nuclease, RNase (Hybridase (registered trademark) Thermostable RNase H, manufactured by epicentre) was used, and as a polymerase, Bst-LF (Bst DNA Polymerase, Large Fragment, manufactured by New England Biolabs) was used.

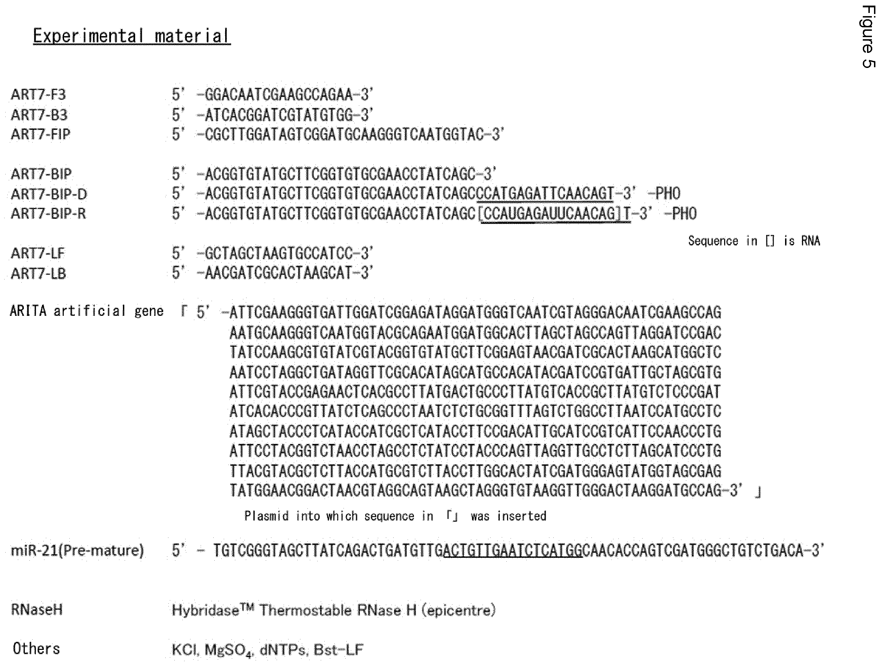

Fig. 5 shows the sequence of each primer used in an experiment for detecting a short DNA.

In Fig. 5, sequence segments complementary between miR-21 and ART7-BIP-D and ART7-BIP-R are underlined.

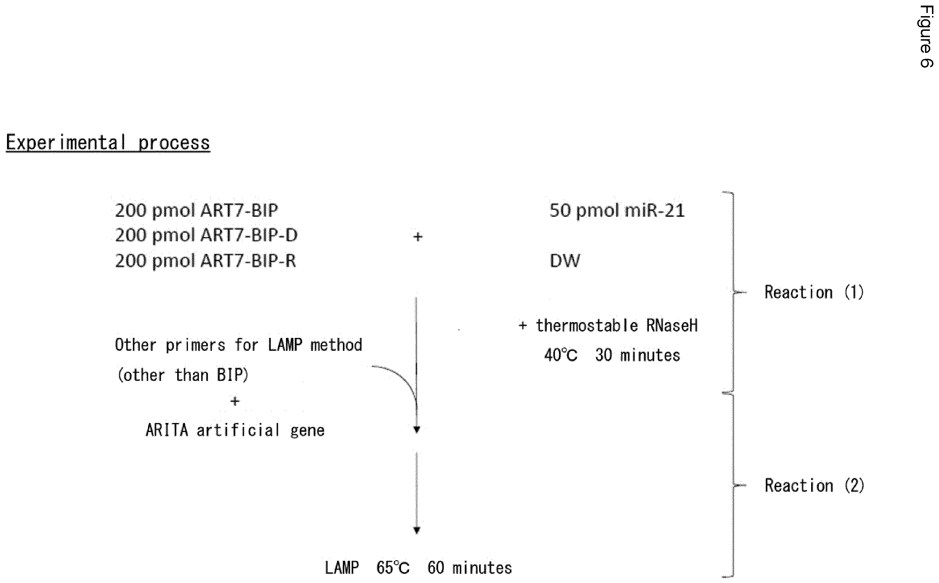

An experimental process for detecting a nucleic acid is shown in Fig. 6.

First, 200pmol ART7-BIP, 200pmol ART7-BIP-D, and 200pmol ART7-BIP-R were each mixed with 50 pmol miR-21, treated with thermostable RNase H, other primers for a LAMP method (ART7-F3, ART7-B3, ART7-FIP, ART7-LF, and ART7-LB) and ARITA artificial genes were then added thereto, and a nucleic acid amplification reaction was carried out by a LAMP method. As a control, not miR-21 but DW (Distilled Water) was used, and mixed with each of ART7-BIP, ART7-BIP-D, and ART7-BIP-R, treated with a thermostable RNase H, other primers for a LAMP method and an ARITA artificial gene were then added thereto, and a nucleic acid amplification reaction was performed by a LAMP method.

In a thermostable RNase H treatment, a reaction solution containing KCl and MgSO₄ was used for enzyme treatment at 40°C for 30 minutes. After the enzyme reaction, an enzyme inactivation treatment was performed at 95°C for 10 minutes.

A nucleic acid amplification reaction of the LAMP method was performed at 65°C for 60 minutes using a reaction solution containing KCl, MgSO₄, dNTPs, and Bst-LF.

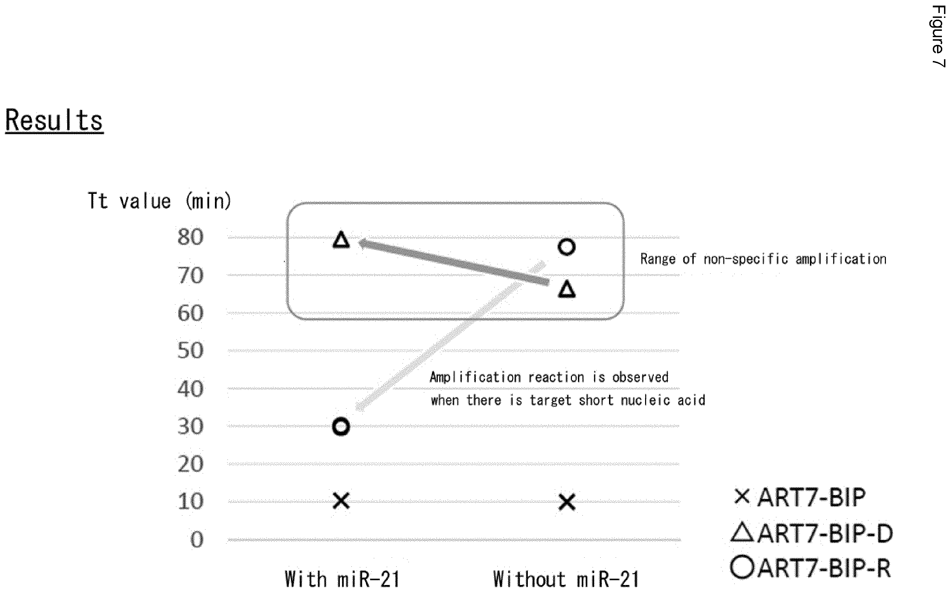

Fig. 7 shows results of measuring the time required for confirming amplification by a LAMP method in detecting a short DNA.

As shown in Fig. 7, in the case of using ART7-BIP-R, which is a primer for nucleic acid detection of the present invention, when miR-21, which is a detection target nucleic acid, is present, a normal nucleic acid amplification reaction occurs, and amplification can be confirmed in a short time, and when miR-21 is not present, amplification time was slow, and no specific amplification was confirmed.

When a normal inner primer ART7-BIP was used, a normal nucleic acid amplification reaction occurred regardless of the presence or absence of miR-21, and amplification could be confirmed in a short time.

When ART-BIP-D including not RNA but DNA as a first polynucleotide segment was used, amplification time was slow regardless of the presence or absence of miR-21, and specific amplification was not confirmed.

### Industrial Applicability

The method for nucleic acid detection, the primer for nucleic acid detection, and the kit for nucleic acid detection of the present invention are useful in industrial fields of clinical tests, clinical test reagents, and research reagents.

### Reference Signs List

- 1: Detection target nucleic acid
- 2: Template nucleic acid
- 201a: Common base sequence
- 3: Primer for nucleic acid detection
- 301: First polynucleotide segment
- 302: Second polynucleotide segment
- 302a: Portion on 3' side of second polynucleotide segment
- 302b: Portion on 5' side of second polynucleotide segment
- 303: Third polynucleotide segment
- 304: Fourth polynucleotide segment
- 4: Nuclease
- 5: Polymerase

## Claims

1. A method for nucleic acid detection for detecting a detection target nucleic acid, using at least
A) a template nucleic acid that is amplified and serves as a detection index,
B) a primer for nucleic acid detection including a first polynucleotide segment having a base sequence complementary to at least a portion of the detection target nucleic acid and a second polynucleotide segment having a base sequence complementary to a portion of the template nucleic acid, wherein
when the detection target nucleic acid is RNA, at least the 5' end of the first polynucleotide segment is DNA, and when the detection target nucleic acid is DNA, at least the 5' end of the first polynucleotide segment is RNA, and
the second polynucleotide segment is linked to the 5' side of the first polynucleotide segment,
C) a nuclease that degrades a DNA-RNA complex, and
D) a polymerase,
the method comprising:
i) contacting a test sample containing the detection target nucleic acid with the primer for nucleic acid detection to hybridize the detection target nucleic acid and the primer for nucleic acid detection to form a DNA-RNA complex,
ii) removing the first polynucleotide segment from the primer for nucleic acid detection by degrading at least a portion of the DNA-RNA complex with the nuclease, and
iii) hybridizing the primer for nucleic acid detection to the template nucleic acid and amplifying the template nucleic acid with the polymerase.

2. The method for nucleic acid detection according to claim 1, wherein
the detection target nucleic acid is a short nucleic acid having a size of from 10 to 100 bases.

3. The method for nucleic acid detection according to claim 1 or 2, wherein
the primer for nucleic acid detection further comprises a third polynucleotide segment including the same base sequence as a portion of the base sequence of a 5' side region from a region complementary to the second polynucleotide segment in the template nucleic acid on the 5' side of the second polynucleotide segment.

4. The method for nucleic acid detection according to any one of claims 1 to 3, wherein
the detection target nucleic acid is RNA, and the nuclease is a nuclease that degrades DNA in the DNA-RNA complex.

5. The method for nucleic acid detection according to any one of claims 1 to 4, wherein
the 3' end of the primer for nucleic acid detection comprises a modification that stops an amplification reaction.

6. The method for nucleic acid detection according to any one of claims 1 to 5, wherein
the primer for nucleic acid detection further comprises a fourth polynucleotide segment that has a non-natural artificial base sequence on the 3' side of the first polynucleotide segment and does not hybridize with the template nucleic acid.

7. The method for nucleic acid detection according to any one of claims 1 to 6, wherein
in the above iii), nucleic acid amplification by a LAMP method or nucleic acid amplification by a PCR method.

8. The method for nucleic acid detection according to any one of claims 1 to 7, wherein
the template nucleic acid is a template nucleic acid having a non-natural artificial base sequence.

9. The method for nucleic acid detection according to any one of claims 1 to 8, wherein
the template nucleic acid comprises a common base sequence identical to a portion of the detection target nucleic acid, and
a portion on the 3' side of the second polynucleotide segment is complementary to the common base sequence.

10. A primer for nucleic acid detection for detecting a detection target nucleic acid, comprising:
a first polynucleotide segment having a base sequence complementary to at least a portion of the detection target nucleic acid, and
a second polynucleotide segment having a base sequence complementary to a portion of the template nucleic acid that is amplified and serves as a detection index, wherein
when the detection target nucleic acid is RNA, at least the 5' end of the first polynucleotide segment is DNA, and when the detection target nucleic acid is DNA, at least the 5' end of the first polynucleotide segment is RNA, and
the second polynucleotide segment is linked to the 5' side of the first polynucleotide segment.

11. The primer for nucleic acid detection according to claim 10, wherein
the first polynucleotide segment has a size of from 10 to 100 bases.

12. The primer for nucleic acid detection according to any one of claims 10 or 11, wherein
the primer for nucleic acid detection further comprises a third polynucleotide segment including the same base sequence as a portion of the base sequence of a 5' side region from a region complementary to the second polynucleotide segment in the template nucleic acid on the 5' side of the second polynucleotide segment.

13. The primer for nucleic acid detection according to any one of claims 10 to 12, wherein
the 3' end of the primer for nucleic acid detection comprises a modification that stops an amplification reaction.

14. In the primer for nucleic acid detection according to any one of claims 10 to 13, wherein
the primer for nucleic acid detection further comprises a fourth polynucleotide segment that has a non-natural artificial base sequence on the 3' side of the first polynucleotide segment and does not hybridize with the template nucleic acid.

15. The primer for nucleic acid detection according to any one of claims 10 to 14, wherein
a portion on the 3' side of the second polynucleotide segment is made not only complementary to a part of the template nucleic acid but also complementary to a part of the detection target nucleic acid.

16. A kit for nucleic acid detection for detecting a detection target nucleic acid, comprising:
A) a template nucleic acid that is amplified and serves as a detection index,
B) a primer for nucleic acid detection including a first polynucleotide segment having a base sequence complementary to at least a portion of the detection target nucleic acid and a second polynucleotide segment having a base sequence complementary to a portion of the template nucleic acid, wherein
when the detection target nucleic acid is RNA, at least the 5' end of the first polynucleotide segment is DNA, and when the detection target nucleic acid is DNA, at least the 5' end of the first polynucleotide segment is RNA, and
the second polynucleotide segment is linked to the 5' side of the first polynucleotide segment, and
C) a nuclease that degrades a DNA-RNA complex.

17. The kit for nucleic acid detection according to claim 16, wherein
the first polynucleotide segment has a size of from 10 to 100 bases.

18. The kit for nucleic acid detection according to claim 16 or 17, wherein
the primer for nucleic acid detection further comprises a third polynucleotide segment including the same base sequence as a portion of the base sequence of a 5' side region from a region complementary to the second polynucleotide segment in the template nucleic acid on the 5' side of the second polynucleotide segment.

19. The kit for nucleic acid detection according to any one of claims 16 to 18, wherein
the detection target nucleic acid is RNA, and the nuclease is a nuclease that degrades DNA in the DNA-RNA complex.

20. The kit for nucleic acid detection according to any one of claims 16 to 19, further comprising D) polymerase.

21. The kit for nucleic acid detection according to any one of claims 16 to 20, wherein
the primer for nucleic acid detection further comprises a fourth polynucleotide segment that has a non-natural artificial base sequence on the 3' side of the first polynucleotide segment and does not hybridize with the template nucleic acid.

22. The kit for nucleic acid detection according to any one of claims 16 to 21, wherein
the template nucleic acid comprises a common base sequence identical to a portion of the detection target nucleic acid, and
a portion on the 3' side of the second polynucleotide segment is complementary to the common base sequence.
